# EUROPEAN PATENT SPECIFICATION

(11) **EP 0 599 850 B1**
(45) Date of publication and mention of the grant of the patent: **10.01.1996**
(21) Application number: 92912632.4
(22) Date of filing: 19.05.1992
(51) Int. Cl.: A61K 9/127

(54) **COMPOSITION AND METHOD FOR TREATING CYSTIC FIBROSIS**
ZUSAMMENSETZUNG UND VERFAHREN ZUR BEHANDLUNG VON ZYSTISCHER FIBROSE
COMPOSITION ET PROCEDE DE TRAITEMENT DE LA MUCOVISCIDOSE

(30) Priority: 16.08.1991 US 745900
(43) Date of publication of application: 08.06.1994
(73) Proprietor: VICAL, INC., San Diego California 92121 (US)
(72) Inventor: FELGNER, Philip, L., Rancho Sante Fe, CA 92067 (US); ABAI, Anna, M., San Diego, CA 92131 (US); MANTHORPE, Marston, C., San Diego, CA 92129 (US)
(74) Representative: VOSSIUS & PARTNER
(86) International application number: US9204225
(87) International publication number: WO9303709

(56) References cited:
- WO-A-90/11092
- WO-A-91/02796
- US-A- 4 897 355
- Cell, vol. 62, 1990, (Cambridge, MA, US), M.L. DRUMM et al.: "Correction of the cystic fibrosis defect in vitro by retrovirus-mediated gene transfer", pages 1227-1233, see the whole document (cited in the application)
- Proc. Natl. Acad. Sci. USA, vol. 84, November 1987, Biochemistry, (Washington, DC, US), P.L. FELGNER et al.: "Lipofection: a highly efficient, lipid-mediated DNA-transfection procedure", pages 7413-7417, see the whole document (cited in the application)
- Proc. Natl. Acad. Sci. USA, vol. 86, August 1989, Biochemistry, (Washington, DC, US), R.W. MALONE et al.: "Cationic liposome-mediated RNA transfection", pages 6077-6081, see the whole document (cited in the application)
- Proc. Natl. Acad. Sci. USA, vol. 87, December 1990, Medical Sciences, (Washington, DC, US), S. SHAK et al.: "Recombinant human DNase I reduces the viscosity of cystic fibrosis sputum", pages 9188-9192, see the whole document (cited in the application)

## Description

The present invention relates to methods for treating cystic fibrosis and in particular to therapies to correct the physiologic defect associated with cystic fibrosis in lung airway epithelia. More specifically, the invention provides a method for directing polynucleotide sequences, protein or a combination thereof to airway epithelia to therapeutically correct the functional cellular defect associated with cystic fibrosis.

Cystic Fibrosis (CF) is an autosomal recessive disease that causes abnormalities in fluid and electrolyte transport in exocrine epithelia. Mutations within the DNA coding for a protein termed the cystic fibrosis transmembrane conductance regulator (CFTR) have been found in virtually all CF patients. Cells of the lung are particularly affected.

In CF, the luminal border of the airway mucosal cell is unresponsive to cAMP-dependant protein kinase activation of membrane chlorine ion channels. The cells are impermeable to Cl⁻, and Na⁺ absorption across the cell membrane is accelerated. Both of these electrolyte imbalances tend to reduce the level of hydration of the airway mucus thus contributing to the viscous lung secretions characteristic of CF. Adventitious bacteria and mycoplasmas enter the lung airways and establish colonies within the mucus. The thick mucus associated with CF isolates these pathogens from the immune system. Since mucociliary clearance is reduced in CF patients, bacterial clearance is also reduced. Lung congestion and infection are thus common. The prolonged presence of these pathogenic agents invariably initiates inflammatory reactions that compromise lung function.

Toxin secretion from bacterial colonies, immune complex deposition and ineffective phagocytosis are believed to result in an imbalance between neutrophil proteinases and their inhibitors. This establishes cycles of prolonged tissue damage.

In healthy lungs, airway epithelial cells slough off slowly over time and are replaced by dividing progenitor cells. The continued tissue damage associated with CF compromises the capacity of the progenitor cells to replace sloughed or damaged airway epithelia. Patients are therefore further predisposed to chronic recurrent infections with its associated tissue inflammation and damage resulting in progressive pulmonary deterioration. Maintenance therapy for CF patients includes mechanical assistance to clear the airways of sputum. Antibiotics are administered either systemically or as aerosols to suppress pulmonary infections. Loss of parenchymal tissue and increased mucoid secretions yield respiratory failure that is additionally complicated by pulmonary hypertension. At present there is no therapy available to directly target the cellular defect associated with CF.

The presence of viscous mucus in CF patients continually presents problems for maintenance therapy. The mucus provides a support and a harbor for bacterial growth. Therefore most antibiotics are applied directly to the lung. Intravenous antibiotics cannot be given in sufficient concentration to inhibit bacterial growth within the mucus without causing adverse secondary effects. The mucus further presents a physical barrier within the lung that prevents dissemination of the antibiotic. In addition, there is some evidence to suggest that some antibiotics specifically bind to components within the mucus further diluting the bacteriocidal effect.

Since the viscous mucus associated with cystic fibrosis restricts antibiotic dispersion in CF lungs, the dispersion of other complexes or compounds within CF lungs may be similarly restricted. Mucus viscosity in CF lungs is in part due to the decreased hydration of the mucus as related to Cl⁻ channel malfunction. However, the mucus from CF patients is additionally thickened by bacteria, purulence associated with aggravated immune reactions and dead epithelial cells. Sputum derived from CF patients has a much higher content of DNA than normal sputum and this DNA further adds to mucus viscoelasticity.

Bovine pancreatic DNase I has been shown to be effective *in vivo* in reducing the viscosity of lung mucus (J. Lieberman, J. Am Med Assoc. 205:312-313, 1968). DNase I therapy was used routinely approximately twenty years ago but is used less often today. These DNase preparations were highly contaminated with impurities that complicated therapy. It might additionally be expected that the repeated use of pure bovine pancreatic DNase could initiate an immune reaction in the lung adding to the pathogenic sequelae associated with CF. Shak et al. recently used recombinant human DNase I to reduce the viscosity of sputum from cystic fibrosis patients *in vitro* (Proc. Natl. Acad. Sci. (USA) 87:9188-9192, 1990. Under prolonged use, human DNase I is expected to be immunologically tolerated over its bovine counterpart. Thus the inhalation of a recombinant human DNase I aerosol may be beneficial to patients with cystic fibrosis for reducing mucus viscosity as compared with bovine DNase I.

Individuals with cystic fibrosis have a life expectancy of 25-35 years. Maintenance therapy alone will not significantly increase the life span of these patients. Human DNase I administration decreases mucus viscosity *in vitro* and may have reduced immunogenicity. Antibiotics help control bacterial colonization. However, none of these therapies directly address the basic pathology of the disease. Thus, there remains an urgent need for a therapy to correct the physiologic defect associated with CF.

Disclosed herein is a novel and heretofore undisclosed therapy for CF that uses DNase I together with a therapy to correct the physiologic defect in the airway epithelial cells. The method has clear advantages over other contemplated therapies for CF and these advantages are discussed in the detailed description of this invention.

### BRIEF DESCRIPTION OF THE FIGURES

Figure 1 is a schematic of a restriction endonuclease map for the human CFTR gene.

Figure 2 is an illustration of the assembly of the CFTR containing construct, pRSV-CF. Figure 2A illustrates the restriction enzymes used to obtain the CFTR gene fragment. Figure 2B is a schematic of the expression plasmid pRSV-lux. Figure 2C is a schematic of the pRSV-CF construct.

Figure 3 illustrates the intracellular delivery of increasing concentrations of protein with a cationic liposome formulation. Figure 3A shows cyclic AMP production in CEM cells as a function of toxin concentration (the points represent the amount of subunit A captured in DOTMA/DOPE MLVs, the squares show the holotoxin, and the empty squares the subunit A in anionic liposomes). Figure 3B shows the corresponding DOTMA concentration on the x axis.

There has been provided in accordance with one aspect of the present invention, a pharmaceutical composition suitable for pulmonary administration comprising therapeutically effective amounts of DNase, a polynucleotide sequence operatively coding for functional protein to remedy the cellular defect associated with cystic fibrosis and an amount of cationic lipid effective to deliver the polynucleotide sequence into pulmonary cells *in vivo*.

Preferably, the amount of DNase is effective to reduce the viscosity of pulmonary mucus in a cystic fibrosis patient thereby facilitating delivery of the polynucleotide sequence to the pulmonary cells. In addition, the amount of polynucleotide is sufficient to encode a therapeutically efficacious amount of protein. This polynucleotide is preferably the cystic fibrosis transmembrane conductance regulator. The polynucleotide is DNA or preferably mRNA and the composition is deliverable by pulmonary instillation or preferably by aerosol.

In accordance with another embodiment of the present invention, the composition comprises DNase, a functional polypeptide to remedy the cellular defect associated with cystic fibrosis and an amount of lipid effective to deliver the polypeptide into pulmonary cells *in vivo* wherein the lipid and polypeptide form a cationic complex. The amount of DNase is preferably sufficient to reduce the viscosity of the pulmonary mucus in a cystic fibrosis patient thereby facilitating delivery of the functional polypeptide into the pulmonary cells. In a preferred embodiment the polypeptide is the cystic fibrosis transmembrane conductance regulator and the composition is suitable for pulmonary instillation and preferably suitable for administration by aerosol.

In accordance with a further aspect of the present invention an apparatus for the treatment of cystic fibrosis is provided comprising a container housing one or more compositions including therapeutically effective amounts of DNase, a macromolecule that provides functional protein to remedy the cellular defect associated with cystic fibrosis, and a lipid suitable for forming a cationic complex with the macromolecule for delivery of the macromolecule into pulmonary cells *in vivo* and means associated with the container to facilitate delivery of the composition to the pulmonary system. The apparatus is adapted to deliver the composition by either pulmonary instillation or as an aerosol. The macromolecule is preferably either a functional polypeptide or a polynucleotide sequence operatively coding for functional protein to remedy the cellular defect associated with cystic fibrosis.

In another embodiment of the present invention a method of treating a patient having cystic fibrosis is disclosed comprising the steps of: providing a battery of active ingredients comprising therapeutically effective amounts of DNase, and effective amount of macromolecule that provides functional protein to remedy the cellular defect associated with cystic fibrosis, and lipid suitable for forming cationic complexes with said macromolecule for delivery of the macromolecule into pulmonary cells in vivo; and delivering the active ingredients to the bronchial passageways of the patient with cystic fibrosis. The treatment is preferably delivered by aerosol and in one embodiment of this method the DNase is administered before delivery of effective amounts of the macromolecule and cationic lipid. The macromolecule and cationic lipid are preferably administered together. The macromolecule may comprise functional protein or polynucleotide sequence operatively coding for functional protein to remedy the cellular defect associated with cystic fibrosis.

In another aspect of this invention a method for treating cystic fibrosis is provided comprising the steps of administering to the bronchial passageways of patients an amount of DNase sufficient to decrease mucus viscosity and delivering to cells of the passageway in conjunction with the DNase an effective amount of macromolecule that provides functional protein to remedy the cellular defect associated with cystic fibrosis and lipid effective to deliver said macromolecule into pulmonary cells *in vivo*. The method may further comprise the step of periodically repeating the treatment until expression of the protein is observed in the pulmonary cells. The macromolecule may comprise polynucleotide sequence operatively coding for functional protein to remedy the cellular defect associated with cystic fibrosis. The polynucleotide may be mRNA or DNA and the mRNA preferably lacks a 5' capping nucleotide. The macromolecule may additionally comprise functional polypeptide to remedy the cellular defect associated with cystic fibrosis and is preferably the cystic fibrosis transmembrane conductance regulator. The DNase is preferably a recombinant protein, and more preferably recombinant human DNase I. The DNase, macromolecule and lipid are preferably delivered via pulmonary instillation or by aerosol.

In a further embodiment of the present invention there is provided a kit useful for treating cystic fibrosis patients comprising one or more containers housing effective amounts of : DNase, a macromolecule that provides functional protein to remedy the cellular defect associated with cystic fibrosis and lipid effective to deliver the macromolecule into pulmonary cells *in vivo*; and means for facilitating the delivery of the DNase, the macromolecule, and the lipid to the bronchial passageways of cystic fibrosis patients. The kit preferably has containers to house predetermined unit dosages of the DNase, macromolecule, and lipid. More preferably, the dosages are unit dosages effective for a single therapeutic treatment.

In a final embodiment, a method for treating cystic fibrosis is provided comprising decreasing the amount of mucus associated DNA in lung passageways; and delivering to the cells of the passageways an effective amount of macromolecule that provides functional protein to remedy the cellular defect associated with cystic fibrosis as a cationic complex effective to deliver the macromolecule into pulmonary cells *in vivo.* The amount of mucus in the lung may be decreased preferably by lung lavage, treating the passageways with an amount of DNase I sufficient to decrease mucus viscosity, or chest percussion and postural drainage. Further the method is periodically repeated until expression of the protein is observed in the pulmonary cells.

The gene responsible for CF has been localized within the human genome. The gene encodes a protein of 1,480 amino acids and is termed the cystic fibrosis transmembrane conductance regulator (CFTR) Its sequence is provided in a publication by Riordan et al (Science 245:1066-1073, 1989.). Individuals with CF have defective CFTR DNA sequences and there are a variety of mutations associated with the CF defect. The most common mutation is a deletion of three nucleotides that encode phenylalanine at position number 508 on the CFTR gene. For CF, a method for delivery of the correct CFTR gene sequence into deficient cells could be used to correct the cellular defect associated with cystic fibrosis.

This invention relates the heretofore undisclosed and unsuggested use of DNase I in combination with lipid-mediated macromolecule therapy for the effective delivery of a therapeutic gene protein or other macromolecule to the respiratory epithelia to correct the cellular defect associated with cystic fibrosis. "Macromolecule" is used herein to describe a polynucleotide, polypeptide or other bioactive molecule. A polynucleotide sequence operatively codes for a polypeptide when it has all the genetic information necessary for expression by a target cell, such as promoters and the like. The polynucleotide can comprise a complete DNA or RNA, gene, a fragment of a gene, or several genes, together with recognition and other sequences necessary for expression. Protein causally related to cystic fibrosis comprises the CFTR protein or polypeptide and peptide fragments thereof, or any other protein or proteinaceous matter shown to have altered cellular levels in cells from cystic fibrosis patients. The enzymatic activity of DNase I is employed not only to decrease sputum viscosity thereby facilitating liposome complex dispersal but to provide unexpected advantages to liposome-mediated macromolecule delivery that are heretofore undisclosed.

### Proposed Gene Therapies for Cystic Fibrosis

The gene therapies for cystic fibrosis that have been proposed to date are primarily virus-based gene therapies. These therapies and complications associated with these therapies are discussed below.

Investigators have introduced the CFTR gene into cells derived from a cystic fibrosis patient *in vitro* to observe the effect that CFTR gene expression has on Cl⁻ transport. Drumm et al. used a retrovirus to carry the CFTR gene into a pancreatic cancer cell line derived from a CF patient (Cell 62:1227-1233, 1990). Stable transformants were assessed for their ability to transport chlorine ions. The addition of a single normal copy of the CFTR gene to a CF cell alleviated the chlorine ion channel defect. These investigators contemplate the use of retroviral vectors for the stable expression of the CFTR gene in CF patients.

Retroviral vectors comprise retrovirus-based gene sequences packaged by retroviral proteins into an infectious virus particle. Typically single-stranded RNA is released into the infected cell. The RNA is reversely transcribed into DNA and this DNA integrates into the host genome. Retroviral-based gene expression requires integration of the gene in question into the cell chromosome before transcription and protein translation can take place. Since the location of gene insertion is not always controlled, there remains the risk of aberrant host cell protein expression, and therefore a risk of cancer. Thus, there have been considerable questions regarding the safety of retroviral gene therapy.

Further, retrovirus gene expression is not always an efficient process. Not all of the airway epithelial cells having the gene defect will be infected by a given aerosolized dose of retrovirus. Only a fraction of those cells that are infected will contain a stably integrated copy of the vector and a still smaller fraction will actually express the functional gene. The process to date is relatively inefficient.

There are other virus-mediated (non-retrovirus) gene delivery systems that are contemplated for lung cell delivery. Adenoviruses and adeno-associated viruses have a natural affinity for the respiratory system. Therefore some investigators believe these viruses may be good candidates for virus-mediated gene delivery in the lung. Adenoviruses, unlike retroviruses, do not require cell division for gene expression; thus they may be more suitable to *in vivo* applications. However, adenovirus and adeno-associated viral gene vectors modelled from natural lung pathogens have the potential for back mutation to a pathogenic state.

As a general rule, all modified virus gene transfer vehicles carry the risk of back mutation and the potential for pathogenic sequelae. Further, virus mediated gene transfer vehicles have inherent size restrictions as to the amount of polynucleotide sequence they can accommodate. In addition, virus gene vectors can only deliver certain types of nucleic acid. Modified nucleic acids, protein and other macromolecules cannot be delivered efficiently using viral vectors. The type of nucleic acid delivered to a cell is restricted to the type of nucleic acid normally packaged by that particular virus. Therefore, RNA is not naturally packaged by a DNA derived virus, and visa versa. Neither RNA nor DNA viruses efficiently package mRNA. A non-virus based gene transfer vehicle for the delivery of macromolecules causally related to CF that would carry less risk to the patent and deliver a range of bioactive molecules would be a significant improvement over currently contemplated therapies.

### Protein delivery to CF Airway Epithelia

It is not always clear at what level damaged or compromised airway epithelial cells such as those found in CF patients could readily express a delivered nucleic acid sequence; be it DNA, RNA or a modification thereof. Thus, it is another object of this invention to introduce protein causally related to cystic fibrosis into the airway epithelium. The delivery of CFTR, for example, to airway epithelia would advantageously correct the chlorine ion channel function in compromised cells, even in those cells compromised to the extent that they are unable to transcribe nucleic acid sequences. Further, the addition of functional CFTR to airway epithelial cells would provide immediate relief to the cell. It is additionally contemplated that protein causally related to CF could be administered not only alone but in combination with nucleic acid. Immediate relief could then be followed by a more extended supply of protein derived from the expression of the delivered nucleic acid sequence. While CFTR seems to be the major defective protein associated with CF, it is possible that with further study, other proteins or macromolecules may be implicated in its pathology. Therefore it is contemplated herein that other macromolecules including other protein and nucleic acid sequences may be similarly administered to the airway epithelia to supplement or regulate an aberrant protein found in CF airway epithelia.

### Lipid-mediated Macromolecule Delivery

Liposomes comprise amphipathic lipids forming hollow lipid vesicles. They have been used *in vitro* as a mechanism for introducing genetic sequences into tissue culture cells (Mannino, R.J. Fould-Fogerite, S., Biotechniques 6:682-690, 1988). Lipids forming liposomes may be positively charged (cationic), negatively charged (anionic) or neutral. Only some lipids fuse with the cell membranes on contact and deliver their associated substances intracellularly. Lipids originally used for liposome mediated gene delivery were not efficient at fusing with the target cell surfaces and were instead taken up by endocytosis. These liposomes were delivered to the cellular lysosome and degraded.

A major advance in liposome development was the discovery that a positively charged synthetic cationic lipid, N-[1-92,3-dioleyloxy)propyl]-N,N,N-trimethylammonium chloride (DOTMA) could interact spontaneously with DNA to form lipid-DNA complexes capable of fusing with negatively charged lipids associated with cell membranes (Felgner, P.L. et al Proc. Natl. Acad. Sci.,(USA) 84:74 13-7417 (1987) denoted in further citations as (PNAS, 1987) and U.S. Patent No. 4,897,355 to Eppstein, D. et al). Liposome-mediated gene delivery, unlike retroviral-mediated gene delivery, can deliver either RNA or DNA. Thus DNA, RNA, a modified polynucleotide or a combination thereof can be introduced directly into the cell cytoplasm (Malone et al. Proc. Natl. Acad. Sci.(USA) 86:6077-6081, 1989).

Lipofectin™ is used *in vitro* for nucleic acid transfection procedures. Lipofectin™ can also be used to introduce foreign polynucleotide sequences into frog and rat cells *in vivo*. Holt et al. introduced reporter genes into neurons from the embryonic brain of Xenopus (Neuron 4:203-214, 1990). Hazinski et al. induced the expression of retrovirus-CAT fusion genes in rat lung by tracheal instillation of Lipofectin-polynucleotide complexes (Am. J. Respir. Cell Mol. Biol. 4:206-209, 1991). However, DOTMA, the cationic lipid component of Lipofectin™ (Bethesda Research Laboratory, Gaithersburg, Maryland), is a diether lipid. These types of chemical structures are not readily degradable *in vivo*. Therefore cationic lipids of this type may not be ideal candidates for human use. Improved cationic lipids described below are better candidates for *in vivo* gene delivery.

The positively and negatively charged lipid vesicles used in the methods of this invention are typically prepared as appropriate from a mixture of either cationic lipids or negatively charged lipids, neutral lipids and cholesterol or a similar sterol. Thus the use of negative, positive and neutral charged lipids or a combination thereof are advantageously contemplated herein. Neutral lipids can be phosphatidylcholine, phosphatidyl ethanolamine, similar phospholipid analogs, or mixtures of these, as well as monoglycerides, diglycerides and triglycerides. The negatively charged lipid reagents of the invention are those comprising at least one lipid species having a net negative charge at physiological pH or combinations of these. Suitable lipid species comprise phosphatidyl glycerol and phosphatidic acid or a similar phospholipid analog.

The terms liposome and lipid vesicles are herein used interchangeably. The term cationic liposome is used herein to denote liposomes containing some quantity of cationic lipid thus permitting them to associate with negatively charged cell membranes irrespective of the amount of negatively charged lipid contained therein. Liposome complex refers to the lipid vesicles associated with bioactive molecules. Cationic complex refers to the association of one or more bioactive substances with lipids such that the charge on the complex is sufficiently positive to permit cell membrane fusion. Thus a cationic complex encompasses a range of formulations that extends from complexes of bioactive molecule with cationic lipid to complexes of bioactive molecules with mixtures of lipid species that include cationic lipid, to cationic protein with neutral lipid. In the complex the macromolecule may be incorporated into the bilayer, complexed to the surface by charge or adsorbed to the surface of the lipid. The lipid reagents of this invention may comprise lipid mixtures similar to that of the physiological cell membrane, comprising phospholipids as primary components. The lipid reagents can further comprise any of the conventional synthetic or natural liposome materials, including phospholipids from natural plant or animal sources such as phosphatidylcholine, phosphatidylethanolamine, sphingomyelin, phosphatidylserine, or phosphoinositol. Synthetic phospholipids that may also be used include, but are not limited to, dimyristoylphosphatidylcholine, dioleoylphosphatidylcholine, dipalmitoylphosphatidylcholine and distearoylphosphatidylcholine and the corresponding synthetic phosphatidylethanolamines and phosphatidylglycerols. Other additives such as cholesterol, glycolipids, fatty acids, sphingolipids, or gangliosides can also be used, as is conventionally known for the preparation of liposomes.

Suitable cationic lipid species comprise known cationic lipids, such as 1,2-bis(oleoyloxy)-3-(trimethylammonio)propane (DOTAP) or N-(w, w-1-dialkoxy) -alk-1-yl-N, N, N-trisubstituted ammonium surfactants, such as DOTMA, or complex cationic lipids having similar structures and properties or mixtures of these. Particularly preferred cationic lipids including those cationic lipids postulated to be more readily degradable *in vivo* include analogs of DORI (DL-1,2-dioleoyl-3-dimethylaminopropyl-β-hydroxyethylammonium) and DORIE (DL-1,2-O-dioleyl-3-dimethylaminopropyl-β-hydroxyethylammonium) as well as DORI ester/ether compounds (DL-1-O-oleyl-2-oleyl-3-dimethylaminopropyl-β-hydroxyethylammonium or DL-1-oleyl-2-O-oleyl-3-dimethyl-aminopropyl-β-hydroxyethylammonium) or other derivatives having the general formula 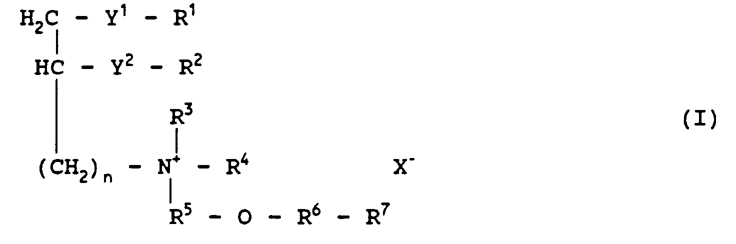
wherein
Y¹ and Y² are the same or different and are -O-CH₂-, -O-C(O)-, or -O-;
R¹ and R² are the same or different and are H, or C₁ to C₂₃ alkyl or alkenyl;
R³ and R⁴ are the same or different and are C₁ to C₂₄ alkyl, or H;
R⁵ is C₁ to C₂₄ alkyl straight chain or branched chain;
R⁶ is -C(O)-(CH₂)ₘ-NH-, a diaminocarboxylic acid which is alkyl, aryl, or aralkyl, or -C(O)-(CH₂)ₘ-NH- linked to said diaminocarboxylic acid, or is absent;
R⁷ is H, spermine, spermidine, a histone, or a protein with DNA-binding specificity, or wherein the amines of the R₇ moiety are quaternized with R³, R⁴, or R⁵ groups; or
R⁷ is an L- or D-alpha amino acids having a positively charged group on the side chain, said amino acids comprising arginine, histidine, lysine, ornithine or derivatives thereof, or the same amino acids wherein the amine of the R₇ moiety is quaternized with R³, R⁴ or R⁵ groups; or
R⁷ is a polypeptide selected from the group consisting of L- or D-alpha amino acids, wherein at least one of the amino acids residues comprises arginine, histidine, lysine, ornithine, or derivatives thereof;
n is 1 to 8;
m is 1 to 18; and
X is a non-toxic anion
Non-toxic anions described herein may be those of pharmaceutically non-toxic acids including inorganic acids and organic acids. Such acids include hydrochloric, hydrobromic, sulfuric, phosphoric, acetic, benzoic, citric, glutamic, lactic acid and the like. For the preparation of pharmaceutically acceptable salts, see S.M. Berge et al., Journal of Pharmaceutical Sciences, 66:1-19 (1977). Further, DOTMA (N-[1-(2,3-dioleyloxy)propyl]-N,N,N-trimethylammonium) could be used in combination with the improved cationic lipids or in combination with cholesterol, lyso lipids or neutral lipids.

In addition to these cationic lipids, other lipids may be added to the cationic lipid of choice. These include but are not limited to Lyso lipids of which lysophosphatidylcholine (1-oleoyllysophosphatidylcholine) is an example, cholesterol, or neutral phospholipids including dioleoyl phosphatidyl ethanolamine (DOPE) or dioleoyl phosphatidyl choline (DOPC). The ratios of lipids may vary to include a majority of cationic lipid in combination with cholesterol or mixtures of lyso or neutral lipids. The molar ratios and preferred combinations of lipid will become apparent from the examples cited below.

Experimental results have indicated that DORI and DORIE are superior to DOTMA as cationic lipid transfection agents and have properties that lend themselves to *in vivo* gene delivery. Further, the activity of DOTMA can be improved by the addition of a hydroxyethyl moiety linked to the nitrogen of the quaternary ammonium. The addition of DOPE to the cationic lipid formulation increases the efficiency of transfection over DORI or DORIE alone. DOPE is more effective than cholesterol at improving transfection efficiency and DOPE at 50 mole% in the cationic lipid mixture is most effective. These optimization criteria will vary for different cationic lipid species.

Polyanionic molecules such as polynucleotides or anionic proteins readily associate with cationic lipids. However the delivery of cationic or neutral proteins to cells requires the use of additional lipid formulations. The use of assembled complexes having a positive charge suitable for spontaneously attaching to negatively charged cell membranes that may comprise neutral or positively charged bioactive substances first encapsulated in or associated with negatively charged lipid vesicles which are next complexed with cationic lipid vesicles having a positive charge before or in the process of administration to the cell. permit the delivery of positive or neutral charged protein causally related to cystic fibrosis. CFTR is a large protein with an amphipathic extracellular face and a cationic cytoplasmic face. The net charge of the molecule is sufficiently positive that a complex of neutral lipid together with CFTR may be able to fuse directly with the cell membrane.

In a positively charged lipid vesicles formulation, the cationic lipid can be present at a concentration of between about 0.1 mole% and 100 mole%, preferably 5 to 95 mole%, and most preferably between 20 and 80 mole%. In a formulation for preparing negatively charged lipid vesicles, the negatively charged lipid can be present at a concentration between about 0.1 and 100 mole%, preferably 1 to 90 mole%, and most preferably 3 to 50 mole%. In order to produce lipid vesicles having a net charge, the quantity of the positively or negatively charged component must exceed that of the alternatively charged component. The alternatively charged lipid can be present at between about 0 to 49 mole% and preferably 0 to 40 mole%.

The neutral lipid can be present in positively or negatively charged lipid vesicles in a concentration of between about 0 and 99.9 mole %, preferably 5 to 95 mole%, and most preferably 20 to 80 mole%. Cholesterol or a similar sterol can be present at 0 to 80 mole %, and preferably 0 to 50 mole%. It is contemplated that the formulations of lipid will vary depending on the bioactive substance to be delivered to the lung.

### DNase I treatment in the Lung

Effective gene therapy requires that the gene be efficiently transported to the affected cells. However we have discovered that the thick mucus that lines the bronchial airways acts as a barrier to the direct delivery of macromolecules into defective bronchial epithelial cells and impedes effective gene therapy.

Experiments begun over 30 years ago indicated that the concentration of DNA in the lung mucus of cystic fibrosis patients is higher than in normal healthy patients. A recent study has indicated that the DNA found in CF patient mucus is almost entirely of human origin (Lethem M.I. et al. Eur. Resp J. 3:19-23, 1990); presumably leukocyte and epithelial cell derived. Bovine pancreatic DNase I decreased the viscosity of CF lung mucus *in vivo* and was approved for human use in the United States in 1958.

Heparin sulfate competitively inhibits cationic lipid mediated delivery of functional gene sequences into tissue culture cells. We believe that DNA, a large polyanionic molecule, inhibits cationic liposome mediated protein or polynucleotide delivery in a similar manner. Thus mucus from CF patients, containing large quantities of DNA, would also have an inhibitory effect. Cationic complex-mediated delivery of CFTR protein or nucleic acid sequences into the bronchial epithelial cells can be reduced or blocked by the presence of extracellular DNA. Example 8 illustrates that polyanions, like DNA, competitively inhibit cationic lipid mediated delivery into tissue culture cells.

The inhibition by the mucus of cystic fibrosis patients would thus be twofold. The mucus creates a physical barrier by preventing the dissemination of a therapeutic agent in the lung and the mucus creates a chemical barrier that is a specific obstacle for cationic complex mediated macromolecule delivery. Therefore cationic complex mediated delivery of bioactive substances in the lungs may have unexpected complications over other cationic liposome or complex mediated delivery procedures directed to other tissues. The use of DNase I in combination with macromolecule therapy directed to the lung is heretofore undisclosed. DNase I may be given prior to or concomitant with gene therapy. Similarly, DNase treatments may need to be repeated until sputum viscosity and the DNA concentration is sufficiently reduced to permit liposomal delivery to the airway epithelial cells. Further, DNase therapy may not be required for each therapeutic treatment.

Bovine pancreatic DNase I is approved for lung therapy in cystic fibrosis patients. However, there are several potential problems associated with its repeated use. The most common problem arises from allergic complications associated with the impurities found in early preparations of the bovine DNase. Contaminating proteases could initiate allergic reactions or directly irritate lung tissue. Pure preparations of bovine pancreatic DNase I may be combined with cationic liposome delivery to the lungs of CF patients.

Even purified preparations of bovine DNase I could initiate an immunologic reaction after repeated applications. Shak et al. have published the sequence for human DNase I and produced a recombinant human DNase I having native DNase I like activity that reduces the viscosity of CF patient sputum *in vitro* (Proc. Natl. Acad. Sci. (USA) 87:9188-9192, 1990). CF patients live on average into their third decade; therefore an immunologically matched DNase I may be more suitable for repeated use and have reduced side effects over its bovine counterpart.

It is also contemplated that other DNA specific exonuclease or endonucleases capable of cleaving DNA *in vivo* and thereby reducing mucus viscosity may be used in the present invention. Purified or recombinant nucleases may additionally be added to preparations of DNase to improve cutting efficiency. For example a combination of DNase I, Lambda-derived exonuclease and Exonuclease III may be employed to increase nuclease activity. Exonucleases and endonucleases chosen should have minimal residual RNase and protease activity, particularly if the contemplated bioactive molecule is RNA or protein. Further, enzymes chosen for DNA digestion should be tested in culture to ensure that the delivery of the bioactive molecule is not compromised by the presence of that enzyme.

While DNase I or other DNA specific nucleases can be used to decrease the viscosity of mucus in the lungs of patients with CF, other enzymes may also advantageously be used to further reduce mucus viscosity. Thus additional enzymes directed to other components of the mucus such as hyaluronidase can be delivered to the lungs prior to or together with the cationic complexes. Since mucus is apparently a barrier for liposome-mediated nucleic acid or protein delivery, the mechanical removal of mucus by lavage, lung percussion, postural drainage or other means can additionally facilitate delivery. These methods could remove sufficient mucus or sufficiently hydrate remaining lung mucus such that DNase I therapy would not be needed. Thus liposome-mediated delivery may be performed alone or selectively combined with either DNase, lavage, or other means to remove the mucus barrier.

As disclosed herein, DNase I therapy has surprising advantages for cationic complex delivery over that which might be expected if DNase I therapy contributed solely to the dissemination of the mucus barrier alone. DNase I therapy, as illustrated in Example 9, increases the transfection efficiency of cationic liposome containing complexes through both physical and heretofore undisclosed chemical means.

The following sections provide examples for the development, production, testing and administration of cationic complexes containing one or more bioactive molecule of choice. For these examples we have chosen to work with the CFTR protein and CFTR nucleic acid. However other bioactive molecules causally related to cystic fibrosis may similarly be employed using similar techniques that would be readily apparent to those of skill in the art in light of this invention.

### Preparation of the DNA construct encoding CFTR

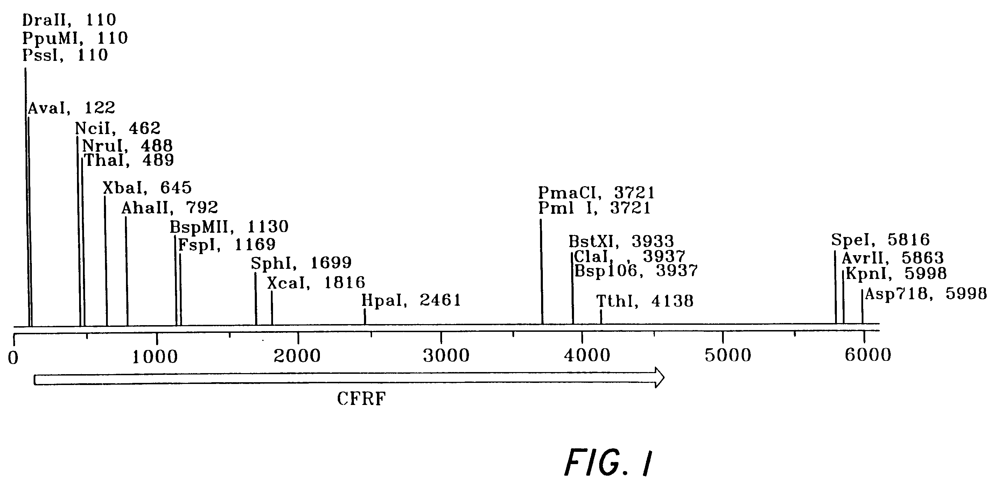

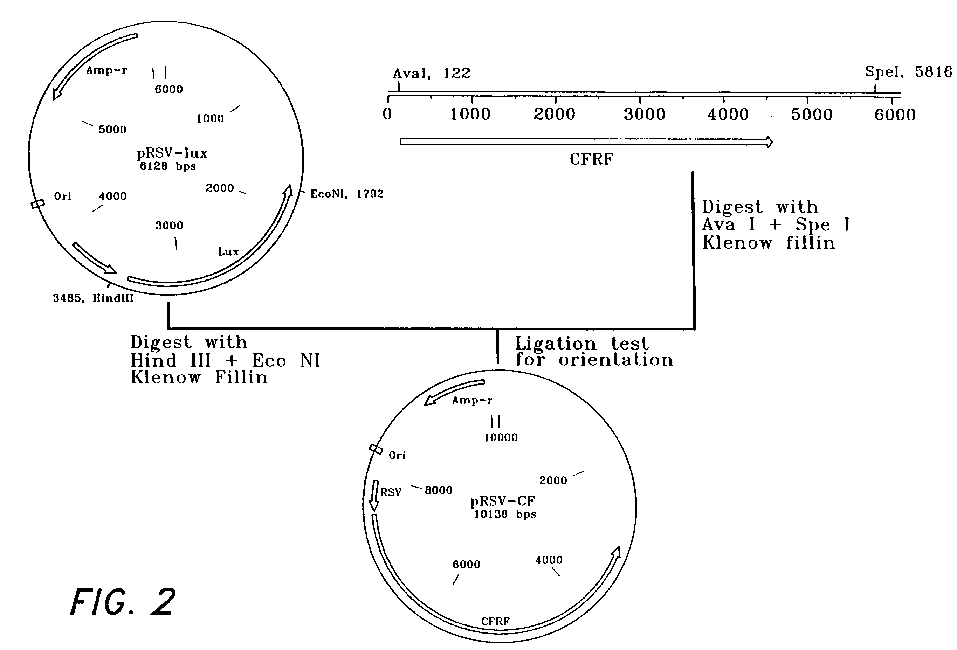

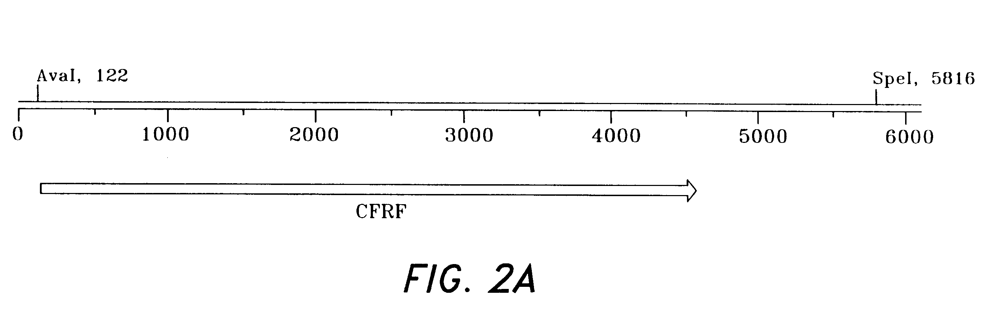

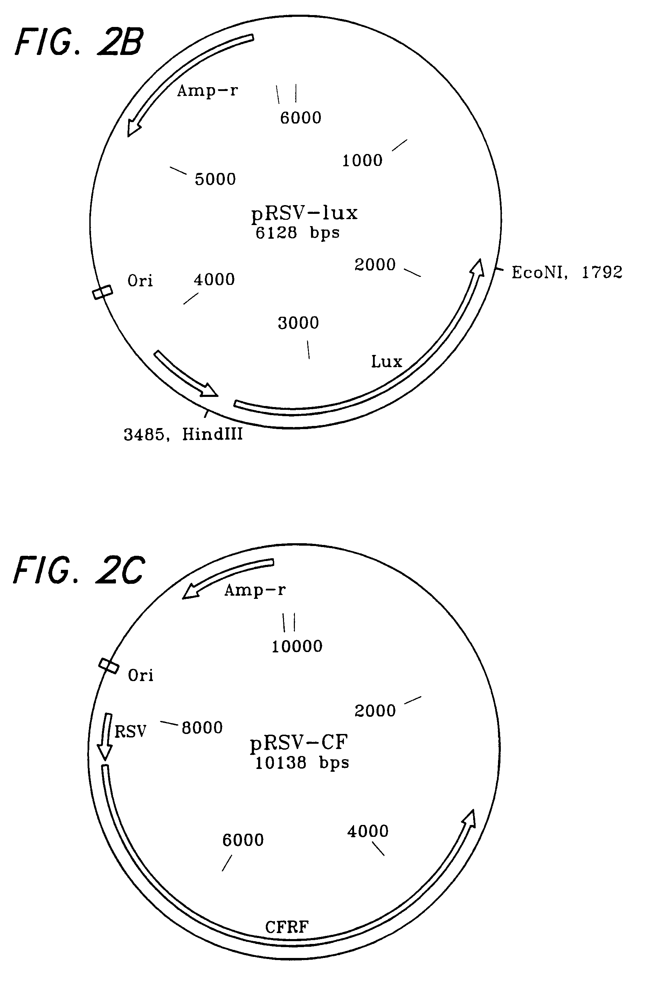

Using cationic lipid-mediated gene delivery, it is possible to introduce the gene encoding CFTR as either DNA or RNA. Figure 1 provides a restriction endonuclease map of CFTR illustrating the Ava I, 122 and Spe I, 5816 restriction sites used for the generation of the CFTR fragment illustrated in Figure 2. Figure 2 illustrates the assembly of the CFTR encoding construct pRSV-CF. Figure 2A is a schematic of the CFTR gene provided as an Ava I, Spe I fragment, 5.7 kb (kilobases) in length. Figure 2B is a schematic of the bacterial expression plasmid pRSV-lux (a gift from Dr. Suresh Subramani, University of California, San Diego). This plasmid contains an ampicillin resistance gene and Rous Sarcoma Virus (RSV) promoter elements. The expression construct containing the CFTR gene is prepared by digesting pRSV-lux with Hind III and Eco RI to remove the luciferase gene fragment. The non-cohesive ends are filled in with the Klenow fragment of DNA polymerase and the vector is purified away from the 1.7 kb fragment. Following Ava I, Spe I digestion of the CFTR fragment, the overlapping ends are similarly filled in with Klenow. Blunt-end ligation is used to join the vector fragment with the insert and positive *E. coli* transformants are screened for inserts having the correct orientation. Figure 2C illustrates the complete plasmid. This description combined with basic molecular biology techniques well known to those with skill in the art permits the re-creation of pRSV-CF. Procedures for all of the methodologies required to complete this and other CFTR constructs as well as other constructs contemplated herein are readily available in molecular biology cloning technique texts such as Molecular Cloning: a Laboratory Manual, Maniatis, T., et al. 1982. Cold Spring Harbor Laboratory, NY denoted in further citations as "Maniatis."

The pRSV-CF construct can be complexed with cationic lipid vesicles to deliver therapeutic amounts of DNA to target cells. For CFTR mRNA production, the CFTR gene fragment is preferably transferred to a second plasmid described below. For both applications, transformed bacteria need to be grown in quantity for plasmid purification. These procedures are described in Maniatis.

It is additionally contemplated that other expression vector constructs containing CFTR could be used in this invention equally well, thus the assembly of vector pRSV-CF is exemplary. Other promoters and control elements, antibiotic resistance markers, or plasmid sequences could be used both for plasmid delivery and to generate mRNA or recombinant protein.

The polynucleotide sequences delivered to the airway epithelia could additionally comprise antisense nuclei acid to regulate or stabilize gene expression. The antisense sequences may bind to either RNA or DNA to upregulate or downregulate gene expression. Antisense sequences delivered by cationic liposomes may bind to a particular gene of interest or to a regulatory region for that gene.

As illustrated in Example 8, the DNA found in cystic fibrosis mucus is expected to block efficient cationic lipid mediated delivery of the CFTR gene in the bronchial epithelial cells. DNase co-administered together with cationic lipid complexes of gene sequences would be expected to hydrolyze the inhibitory DNA present in the mucus. However, the DNA would also digest therapeutic DNA sequences in the formulation. In order to permit the co-administration of the CFTR gene with DNase, the pH of the excipient maybe raised or lowered to a pH that is inhibitory to DNase. DNase would then be activated only when delivered to the lung. Further, DNA may be modified to render it more resistant to DNase digestion. For example, if circular DNA is used, an exonuclease could be added to the lung such that linear DNAs would be preferentially degraded.

In accordance with another embodiment of the invention wherein the delivered polynucleotide is DNA, DNase may be administered 2-4 hours before gene delivery. Further, residual DNase activity can be monitored by standard laboratory means prior to gene therapy. Other methods including postdural drainage and lavage are disclosed herein as alternatives to DNase therapy to reduce the viscosity of the lung mucus. It is additionally possible to lavage the lung following DNase therapy before administering DNA, thereby reducing the inhibitory effects of the DNase on the polynucleotide. Unless the DNA can be modified and made resistant to DNase digestion, or unless the DNA is minimally exposed to DNase, mRNA is preferred to DNA.

### Preparation of RNA

In general, a RNA based CF therapy would be expected to pose fewer regulatory problems than a DNA based therapy, since in the absence of reverse transcriptase, RNA sequences have no theoretical way to integrate into the host genome. Although the duration of expression from mRNA transfected sequences is shorter than for similar DNA sequences, this does not represent a problem for CFTR gene therapy in the lung. Bronchial epithelial cells naturally turn over and this turnover may be more rapid in CF patients. Therefore the potential advantage of longer term DNA expression from these cells may be lost as a result of cell turnover. As will be discussed in Example 12, the contemplated aerosol route of administration for this therapy is easily performed on a daily or even twice daily basis, therefore the need for an extended duration delivery system is less essential than for gene therapies directed to other tissues.

To prepare mRNA, from this example, the CFTR construct is transferred to a second plasmid pSC-CFTR-2 containing the T7 RNA polymerase promoter. The construction of this plasmid is described by Gregory et al (Nature 347:382-386, 1990). The plasmid is linearized with Ava I and aliquots of linearized DNA are combined with the appropriate nucleotides, an RNase inhibitor, T7 RNA polymerase, dithiothreitol, and the required Tris-HCl buffer. The procedure is well known to those with skill in the art and is outlined in detail in Maniatis. Once mRNA is prepared it may be necessary to add a 5' terminal base (usually a guanine) to the RNA molecule. In the cell cytoplasm this is performed by a guanylyl transferase. This "5'cap" imparts stability to the mRNA.

*In vitro* transcription reactions employ one of two methods to add the 5' cap to the transcribed product. The cap analogue may be directly incorporated into the RNA during the transcription reaction or, following transcription, guanylyl transferase is added to transfer the 5'terminal cap to the mRNA transcripts. Both procedures are costly and relatively inefficient. Residual uncapped message is unstable and can degrade either during preparation of the lipid/nucleic acid complex or inside the cell before translation can take place.

An alternative to the 5'cap structure has been used with some success. Some members of the picornavirus family including Encephalomyocarditis virus (EMV) have mRNAs that are naturally uncapped, yet they are translated and stable. A 650 bp (base pair) sequence in the 5' untranslated region provides a ribosome binding site that permits ribosome binding without a capping structure (Elroy-Stein et al. Proc. Natl. Acad. Sci. (USA) 86:6126-6130, 1989). This sequence is placed just downstream of the promoter sequence and can enhance expression 5 to 10 fold. Use of this sequence permits the generation of stable mRNA with reduced manufacturing costs.

In addition, other regulatory sequences can be added to increase stability of the transcript or to improve the translational efficiency of the RNA. Exonuclease-resistant RNAs such as circular mRNA, chemically blocked mRNA, and mRNA with a 5' cap are preferred, because of their greater half-life *in vivo*. One preferred mRNA is a self-circularizing mRNA having the gene of interest preceded by the 5' untranslated region of polio virus. It has been demonstrated that circular mRNA has an extremely long half-life (Harland et al. Development 102: 837-852, 1988) and that the polio virus 5'untranslated region can promote translation of mRNA without the usual 5' cap (Pelletier et al. Nature 334:320-325, 1988). The present invention could also include the use of mRNA that is chemically blocked at the 5' and/or 3' end to prevent access by RNase. Such chemical blockage can substantially lengthen the half-life of the RNA *in vivo*. Such agents include C2 AminoModifier and Amino-7-UTP (Clonetech, Palo Alto, CA). Additional regulatory sequences can be added to the DNA construct to improve the RNA yield in transcription reactions. Examples of positive sequence motifs are the translational initiation consensus sequence identified by Kozak (Nucleic Acids Res. 15:8125, 1987) and 5' UTR sequences that act to enhance translation activity (Hentze et al., Proc. Natl. Acad. Sci. (USA) 84:6730, 1987 and Klemenz et al. EMBO J. 4:2053, 1985)

In a preferred embodiment for the delivery of nucleic acid sequences to bronchial airway cells, mRNA containing the EMV sequence derived from plasmid pSC-CFTR-2 is complexed with cationic liposomes using a preferred formulation as outlined in Example 2 and the section entitled Liposome Formulation. These complexes are delivered to bronchial airway cells as an aerosol alone or with DNase I. These complexes may additionally be delivered alone or in combination with liposomes containing the CFTR protein. It is contemplated that mixtures will be prepared on an individual basis depending on the clinical status of each patient.

We estimate that a 1000 liter fermentor will yield approximately 10 grams of purified gene. The mRNA copy of this gene can be produced enzymatically *in vitro* with at least a 100 fold amplification to produce 1 kg of mRNA product.

CFTR encoding mRNA transfection has a number of benefits over DNA transfection for *in vivo* applications. Once in the cell cytoplasm the mRNA is intended for immediate translation. Since the nucleic acid sequence is not part of the replicatory machinery of a virus and is not designed to integrate into the host genome, the mRNA is safer to prepare and use. Furthermore, mRNA gene therapy has no lasting deleterious effect on the cell. Over time, the mRNA will degrade and protein translation will decrease. This therapy is particularly suited to the lung where there is a natural turnover of target cells and where repeated therapies are expected.

An exemplary unit dosage composition would include from about from about 0.25 ml to about 25 ml total volume in phosphate buffered saline (PBS) or other suitable physiologically acceptable carrier suitable for bronchial instillation. Preferably, the composition includes from about 0.1 to about 20 mg/ml mRNA or DNA complexed with sufficient lipid to provide a mole lipid to mole macromolecule ratio of about 1 x 10² to about 1 x 10⁶ lipid molecules per mole protein and more preferably a ratio of about 1 x 10³ to about 1 x 10⁵ lipid molecules per mole protein. This formulation may itself contain 3 ug/ml DNase I (between about 1.0ug/ml to about 10mg/ml) or equivalent. Alternatively, the DNase may be administered separately, preferably simultaneously with or up to 4 hours prior to the polynucleotide delivery.

### Preparation of CFTR protein

The CFTR protein can be produced directly from the construct outlined above. Purified plasmid is transfected into chinese hamster ovary cells (CHO), HeLa or other suitable eukaryotic tissue culture cells. Similarly the CFTR protein can be produced in a bacterial or baculovirus systems. The method of expressing a given protein will depend on a number of variables that include but are not limited to; the size of the protein, solubility, where the protein is normally found in the cell, the quantity and purity of protein that is required, its charge and whether or not post-translational modifications are required for protein function. Thus the methods chosen for protein preparation are determined by the individual characteristics of the desired protein.

CFTR is a rather large integral membrane glycoprotein with two transmembrane domains each consisting of six hydrophobic helices. Overall, very little of the entire protein is exposed to the exterior surface. In addition the CFTR molecule appears to carry an excess of positive charges.

The isolation and purification procedures for integral membrane proteins are different from procedures for the isolation and purification of cytosolic proteins. The extensive contact between the hydrophobic transmembrane sequences and the apolar core of the membrane lipid bilayer makes purification somewhat more difficult. For these types of proteins purification requires that the lipid bilayer be dissolved with detergents or organic solvents. Once solubilized, these integral membrane proteins precipitate in simple aqueous buffers. Long-term aqueous solubility requires stronger and harsher detergents such as SDS. These harsher procedures are more likely to cause protein denaturation. Once the cell lipids are dissolved away integral membrane proteins such as CFTR can be incorporated or reconstituted into artificial membranes. These complexes are termed proteoliposomes. These procedures are reviewed by Cornelius et al. (Biochim. et Biophys. ACTA 1071, 19-66, 1991).

The membrane protein of interest is isolated from its native membrane environment with the aid of detergents and separated from other membrane constituents by centrifugation. The crude membrane preparation can be treated with mild SDS treatment to extract impurities using controlled detergent concentration, temperature, duration, pH and ionic strength. Further purification can be achieved by fractionation with gradient, zonal centrifugation, or affinity chromatography if applicable. Solubilization is then employed both to obtain further purification and to form a stable protein preparation. The solubilizing detergent binds to and shields the hydrophobic part of the membrane protein, frees it from the original membrane lipids, and makes it accessible to further purification as a water-soluble protein.

Bilayer forming lipids are re-added to the protein. These lipids serve both to stabilize the protein and to act as a solvent. The proteins are co-solubilized with the lipids with either the same or different detergents. Examples of such detergents include Triton X-100, octyl glucoside and C1288 (available from Sigma Chemical Co., St. Louis, MO). The detergents are removed from the mixed micelles which results in the spontaneous formation of proteoliposomes. Fast and reproducible preparation of homogenous unilamellar vesicles by controlled dialysis can be achieved using a Liposomat (MM Developments, Ottawa, Canada). Alternatively, non-ionic detergents can be removed with Bio-beads (BIO-RAD, Richmond, CA). Detergents adsorb to the polystyrene beads which can be added batch-wise and removed by centrifugation, filtration, or by chromatographic techniques.

The CFTR protein can be produced directly from the pS-CFTR-2 derived CFTR mRNA or from transfected cells. Purified plasmid is transfected into chinese hamster ovary (CHO), HeLa or other suitable eukaryotic tissue culture cells. The membrane bound protein is isolated by immunoaffinity chromatography of the cell lysate. A bacterially produced protein can presumably be purified by the method of Oesterhelt and Stoeckenius (Methods of Enzymology, 31: 667-678, 1974).

The relatively pure but still membrane bound protein originating from either of the above expression systems is solubilized with octyl glucoside, Triton X-100 or C12E8, at optimal detergent:protein molar ratios determined empirically(a starting point is 120-12,000 detergent molecules per protein molecule). The choice of lipid or lipids (neutral:PCs, fusogenic:PEs, cationic:DORI, DORIE, DOTMA, etc or a combination of these) and the choice of detergent (one of the above) is dried down from organic solvent (for example ethanol) on the wall of a glass vial in a rotary evaporator, the film is evacuated overnight, and subsequently solubilized by the chosen aqueous buffer. The protein and the lipid solutions are then mixed above the phase transition temperature of the lipids allowing ample time for homogenous mixed micelles to form. The subsequent removal of the detergent can be done using Liposomat or Bio-beads.

Membrane fusogenic "self-delivering" liposomes for CF treatment are thus formulated utilizing the net positive charge of the CFTR protein together with a careful choice of carrier lipids (such as DOTMA/DOPE, DORI/DOPE DORIE/DOPE or DOPE alone stabilized by the association with the membrane protein). In a preferred formulation DORI/DOPE lipids are combined with purified CFTR in TritonX-100.

Additionally, the CFTR protein can be produced by translation in a rabbit reticulocyte lysate system with microsomal membranes and 0.5% Triton X-100 detergent present, from RNA transcribed in vitro by RNA polymerase (Gregory RJ et al, Nature 347:382-386, 1990).

There are a variety of constructs, methods of preparation and methods of purification for DNA, mRNA and protein. Inclusion of all such examples is not considered necessary to convey the spirit of this invention. Those with skill in the art will readily appreciate that a variety of constructs could be employed to produce similar results.

### Liposome Formulation

For a detailed review of methodologies relating to liposome preparation see Liposome Technology by Gregoriadis (CFC Press, NY 1984), Liposomes by Ostro (Marcek Dekker, 1987), and a review by Lichtenberg,et al. (Methods Biochem. Anal. 33:337-462, 1988).

The first step in cationic lipid mediated transfection or polyanion delivery involves the spontaneous formation of a complex between cationic lipid vesicles and the polyanionic macromolecules. This step is mediated by strong, cooperative ionic interactions between the positively charged groups on the lipid vesicles and, for example, the negatively charged phosphate groups on the DNA or RNA. When sufficient quantity of cationic lipid is used, the resulting complexes have a net positive charge and therefore will attach spontaneously to cell surfaces. Cell surface attachment is followed immediately by fusion of the cellular and liposomal membranes thus allowing material in the complexes to escape degradation from lysosomal compartments permitting direct delivery into the cytoplasm.

The active component of these fusogenic liposomes is a bilayer forming synthetic cationic lipid, such as DORI, DORIE or DOTMA. The fusogenic Capacity of these vesicles as well as the complexes they form can be somewhat controlled by the choice of neutral lipid-primarily and preferentially DOPE-coincorporated at different molar ratios into the liposome membrane. Alpha-tocopherol (Sigma, St. Louis, MO) can be added to the lipid bilayer to prevent oxidative degradation. Formulation buffers need to be of low ionic strength to avoid shielding of the charges. Formulation methodologies that result in unilamellar vesicles of the smallest possible size are preferred in order to provide the largest possible cationic surface to ensure that most efficient complexation with polyanionic macromolecules or anionic liposomes and subsequent fusion with and delivery to the target cells. Sonication or microfluidization are well described liposome formulation methodologies that fulfill these criteria. The preparation of sonicated cationic liposomes is described by Felgner, et al. (PNAS, 1987) and Example 1.

Nucleic acids, polyanionic proteins and other macromolecules can be complexed directly with cationic lipid vesicles as illustrated in Example 2. However, since polyanions represent only a subset of bioactive molecules, other lipids or techniques must be used to prepare deliverable cationic complexes suitable for neutral or cationic molecules. Small polar or hydrophilic molecules such as peptides, small proteins (enzymes), drugs etc. can be encapsulated into neutral, negatively charged or positively charged liposomes. Larger cationic proteins can be directly complexed with neutral lipid or sequentially complexed first with anionic lipid followed by cationic lipid. Lipophilic or amphipathic molecules can additionally intercalate into the lipid bilayer of cationic vesicles.

Encapsulation or incorporation of bioactive molecules into cationic liposomes enables their "direct" delivery to the membranes or the cytoplasm of target cells, similarly to transfection.

The cationic lipids of the invention are formed into vesicles according to methods for liposome formulation published in the literature and known to those with skill in the art. The applicable liposome technologies are those that are non-degrading for the biomolecules, ensure high capture, and result in a vesicle population uniform in size. Examples of such procedures include the reverse-phase evaporation procedure of Wilshut, J. et al. (Biochemistry 19:6011-6021, 1980), freeze-thaw extrusion according to Mayer, L. et al. (Biochim. et Biophys. ACTA 858:161-168, 1986), various detergent dialysis methods (Liposomat), simple repeated freeze/thaw procedures followed by hydration. In all of these methods, lipophilic or amphipathic molecules are codissolved with the lipid constituents of the liposomal formulation in organic solvents or with the aid of detergents. Polar or hydrophilic molecules dissolved in the aqueous formulation buffer are applied directly to the dry lipid film. Specific formulations are found in Examples 3, 6, and 7.

Bioactive molecules that will not encapsulate or incorporate directly into cationic lipid vesicles, may incorporate negatively charged liposomes.

Traditional liposomes with zero or a net negative surface charge have been shown to enter tissue culture cells primarily via receptor mediated endocytosis. They are internalized via coated pits which subsequently deliver them to the lysosomal compartment of the cell where they are degraded.

Vesicles presenting a net negative surface charge can be complexed (coated) with a quantity of small positively charged vesicles. The quantity of positively charged vesicles added to the negatively charged vesicles or primary complexes should be sufficient to produce liposome-liposome complexes with a net positive surface charge that is sufficient to encourage their attachment and fusion to the target cell surface. The fusion event between cellular and liposomal (and possibly between cationic-anionic liposomal) membranes allows the lipophilic or amphipathic molecules to become incorporated into cellular membranes and the polar or hydrophilic molecules to be delivered into the cytoplasm. Theoretically, the number of positive charges contributed by the positively charged liposomes should be in excess over the negative charges contributed by the negatively charged lipids and the protein or other molecule carried by the anionic vesicles.

This method provides a vehicle for bioactive molecules primarily associated with anionic liposomes to indirectly utilize the advantageous cationic lipid mediated intracellular delivery route thus avoiding lysosomal degradation. The anionic lipids of choice (DOPG) together with neutral lipids (dioleoyl phosphatidyl choline, dioleoyl phosphatidyl ethanolamine, cholesterol or sphingomyelin) are formed into vesicles according to methods for liposome formulation published in the literature and known to those skilled in the art. The applicable liposome technologies are similarly those that are non-degrading for the biomolecules, ensure high capture, and result in a vesicle population uniform in size.

Polynucleotide/cationic lipid complexes are prepared by suspending the polynucleotide in sterile water or low ionic strength solution (10% sucrose, 5% sorbitol or 5% glucose) to create a isotonic solution by mixing 0.5 ml of a 0.5 to 1 mg/ml polynucleotide solution with 0.5 ml of sonicated liposome mixture at 40-100 ug/ml. The lipid reagent is placed in a polystyrene tube and the DNA solution is added to the lipid with a gentle vortex. Ideally equal volumes of DNA and lipid are added with the cationic lipid monomer to nucleotide monomer ratio of 0.5 to 100. These methods are essentially as described in Felgner, P.L. (PNAS,1987). and Felgner, P. and M. Holm, Focus, 11(2) Spring, 1989. The concentration of polynucleotide in solution can be from about 0.01 µg/ml to 50 mg/ml, preferably from 1 µg/ml to 10 mg/ml and most preferably from 10 µg/ml to 1 mg/ml. The concentration of positively charged lipid vesicles can range from between 0.1 µg/ml to 100 mg/ml, preferably from 1 µg/ml to 100 mg/ml, and most preferably from 10 µg/ml to 50 mg/ml. The solutions may be mixed together in a low ionic strength buffer, that is, having an ionic strength less than that of 25 mM sodium chloride. Sorbitol, sucrose or glucose can be used to render a low ionic strength buffer isotonic. Adsorption of the polyanionic polynucleotides to the cationic vesicles reduces the negative charge character of the polynucleotides. The ratio of positive to negative charges in the polynucleotide/cationic lipid complexes may be from about 100:1 to 0.1:1 and preferably 20:1 to 0.2:1.

In a preferred polypeptide/lipid formulation 1 x 10² to 1 x 10⁶ lipid molecules are added per mole of protein and more preferably 1 x 10³ to 1 x 10⁵ lipid molecules are added per mole protein. For cationic proteins such as CFTR, DOPC or saturated versions thereof, sphingomyelin, PC, PE and combinations of PE and PC are preferable lipids for the formulation of cationic complexes. For cationic complexes comprising polyanionic molecules and an amount of cationic lipid to facilitate cell membrane fusion; example 6 provides preferred formulations of DOPG and DOTMA, example 8 and 10 provides preferred formulations for DORIE:DOPE,

According to a preferred method, the component lipids are dissolved in a solvent such as chloroform and the mixture evaporated to dryness as a film on the inner surface of a glass vial. On suspension in an aqueous solvent, the amphipathic lipid molecules assemble themselves into primary liposomes. If other molecules are present in the aqueous solvent, such as, for example, a bioactive substance, these will be captured within the liposomes, as indicated in Examples 2 and 3. Otherwise, empty liposomes will be formed, as in Example 1. These primary liposomes are reduced to a selected mean diameter by means of the freeze-thaw and extrusion procedure previously described.

A hydration solution is added to the dried lipid film to form primary liposomes. The hydration solution can be any biologically compatible buffer solution comprising isotonic saline (0.9% NaCl) or phosphate buffered saline, or low ionic strength buffers comprising 5% sorbitol or 10% sucrose in 10mM Tris, pH 7.4. Such buffers are well known to those skilled in the art. The concentration of the bioactive substance in the hydration buffer which is intended for intracellular delivery can vary widely depending on the substance or the application; this concentration can be between 1 picogram/ml and 500 mg/ml. Following hydration of the lipid film, the resulting liposome suspension can be further processed by any one of a number of procedures; for example, the sample can be forced through Nucleopore™ membranes to produce vesicles of a size comparable to the pore size of the membranes.

For delivery of polyanionic protein or nucleic acid, the bioactive molecules are complexed directly with cationic lipid as illustrated in Example 2. In an alternate approach, termed indirect encapsulation, the macromolecules intended for intracellular delivery are first encapsulated into negatively charged liposomes, or complexed with empty negatively charged liposomes. These primarily negatively charged complexes are then complexed with a quantity of positively charged lipid vesicles. Immediately upon mixing the two solutions containing the oppositely charged lipid vesicles, complexes form spontaneously to produce cationic liposomes.

The quantity of positively charged vesicles added to the encapsulating negatively charged vesicles should be sufficient to encourage attachment of the complexes to the target cell surface. In addition a positively charged protein can be covalently attached to a cationic lipid. Accordingly, the ratio of positive to negative charges in the final complexes may be from about 100:1 to 0.1:1 and preferably 20:1 to 0.2:1.

### EXAMPLE 1: Preparation Small "Empty" Sonicated Cationic Liposomes

Lipids used: DORI, DORIE or DOTMA and DOPE, at 1:1 molar ratios. Hydrating buffer: sterile deionized water or a low ionic strength biological buffer (for example 10 mM pH=7.4 TRIS) made isotonic with sorbitol, sucrose or glucose. Anti-oxidant: alpha-tocopherol, 0.15 mol % of the total lipid.

5 micromoles of DOTMA, DORI or DORIE is combined with 5 micromoles of DOPE and 0.015 micromoles of alpha-tocopherol in about 0.5 ml of chloroform in a small glass vial, and the lipid mixture is dried down in a rotary evaporator (Buchi Rotavapor, Brinkmann, Switzerland) or under gentle nitrogen stream followed by overnight evacuation under a vacuum pump to remove traces of solvent. The thin lipid film is then hydrated in the capped and sealed vial with 0.5 ml of the buffer of choice to form multi lamellar vesicles (MLVs), vortexed, and finally sonicated to clarity (for about 10-20 minutes) in a Model 350 sonicator (Heat Systems-Ultrasonics, Farmingdale, NY), at 15° C.

### EXAMPLE 2: Complexation of Polynucleotides or Anionic Liposomes with Small Empty Cationic Liposomes

Immediately before use, 0.5 ml of 1mg/ml mRNA or DNA solution or anionic liposome suspension is added to 0.5 ml of small "empty" cationic lipid suspension (such as in Example 1) in a polystyrene test tube and gently vortexed. The molar ratio of the anionic or the nucleic acid base unit to cationic lipid ratio is 1:5. Both liposome suspensions are freshly diluted from their concentrated stock solutions to the desired concentrations (e.g. 100 micromolar DOTMA and 20 micromolar DOPG, to result in a mixture of 50 nanomoles of DOTMA and 10 nanomoles of DOPG in 1 ml, etc.), with their formulation buffers, or in case of tissue culture application, with serum free OPTI-MEM (GIBCO, Bethesda, MD).

### EXAMPLE 3: Anionic and Cationic Liposomes Labelled with Rhodamine-PE in the Lipid Bilayer

A mixture consisting of 5 micromoles of POPC (1-palmitoyl-2-oleoyl-phosphatidyl choline), 2 micromoles of DOPG, 3 micromoles of cholesterol, 0.05 micromoles of Rhodamine-PE and 0.015 micromoles of alpha-tocopherol was dried down from a 0.5 ml chloroform solution into a small glass vial in a rotary evaporator, and further evacuated overnight with a vacuum pump. Similarly, a mixture of 5 micromoles of DOPE, 5 micromoles of DOTMA, DORI or DORIE, 0.05 micromoles of Rhodamine-PE and 0.015 micromoles of alpha-tocopherol was dried down from a 0.5 ml chloroform solution into a small glass vial in a rotary evaporator, and further evacuated overnight with a vacuum pump. The dry lipid vials were hydrated with 0.5 ml 10 mM pH=7.4 TRIS/sorbitol buffer, followed by sonication as described in EXAMPLE 1.

Alternatively, these formulations can be freezed/thawed as described above, with or without the inclusion of multiple extrusion cycles through polycarbonate membranes of a poresize of choice (50, 80, 100 or 200 nm) in an Extruder (Lipex Biomembranes Inc., Vancouver, Canada).

### Demonstration of fusogenicity of cationic liposomes in vitro

Conventional liposomes, formulated primarily of neutral and/or negatively charged phospholipids, have been shown to enter tissue culture cells via receptor mediated endocytosis. They are internalized via coated pits which subsequently deliver them to the lysosomal compartment where they are degraded.

The behavior of the lipid vesicles is visualized by epifluorescence microscopy. CV-1 cells (African green monkey kidney, obtained from ATCC) are exposed to fluorescently labelled liposomal formulations using preparation methods outlined in Example 3. Vesicles delivered to the lysosomal compartment show a distinct punctate appearance, while vesicles fusing with cellular membranes result in diffuse staining. The intracellular localisation of these vesicles can be pinpointed further. When cells are pre-treated with 8-hydroxy-1,3,6-pyrenetrisulfonic pyranine (HPTS), the lysosomotropic green fluorescent dye will highlight the lysosomal compartment of the cell. By incorporating rhodamine phosphatidylcholine (N-(lissamine rhodamine B sulfonyl) phosphatidylethanolamine), into lipid vesicles, co-localization with HPTS into lysosomes can be readily distinguished from delivery into other intracellular sites. The green (HPTS) and red (Rh-PE) fluorescence can be viewed and photographed consecutively with violet and green filter blocks respectively. The general cell appearance is visualized with differential interference contrast (DIC) microscopy. Neutral or anionic lipid vesicles deliver the Rhodamine-PE probe to the lysosomal vesicle and produce a distinct punctate appearance. These lipids are not useful in delivering substances to the cytoplasm or to cell membranes. The Rhodamine-PE membrane probe is used to label cell membranes following exposure to DORIE containing vesicles. The diffuse fluorescent pattern results from fusion of the vesicles with the plasma membrane and with intracellular membranes. This appearance is dramatically different from the distinct punctate patterns of lysosomally localized HPTS and negatively charged lipid vesicles.

### EXAMPLE 4: Cationic liposomes deliver fluorescent lipid to cell membranes

Delivery of CFTR nucleic acid sequences to the cell requires fusion of the liposome with the cell surface and release of the liposome contents into the cell cytoplasm where the transcriptional and translational machinery of the cell can process the nucleic acid. However, for the effective delivery of CFTR protein, the protein must be inserted into the cell membrane in the correct orientation. To test the ability of the liposomal delivery system to deliver a membrane protein to the cell membrane, DORIE vesicles were prepared as in Example 3 with a trace amount (0.5M%) of Lissamine rhodamine B sulfonyl phosphatidylethanolamine (Avanti Polar Lipids, Birmingham, AL). The fluorescent tag coupled to phosphatidylethanolamine is a membrane probe that simulates protein visualization in the targeted cell. Thus this fluorescent membrane label is representative of the CFTR integral membrane protein which is similarly anchored into a membrane bilayer in its native configuration.

Results showed staining of cell surface membranes. No punctate cell staining was observed indicating that the majority of the liposomes were associating with the cell surface and not with the lysosomes.

In the control experimental set, Rh-PE labelled anionic vesicles were delivered to CV-1 cells already stained with HPTS. CV-1 cells were treated in OPTI-MEM as disclosed by Felgner et al.(PNAS, 1987). 5 X 10⁵ cells were exposed to 2nmol DOPG labelled with Rh-PE in the lipid bilayer for 1h. at 37°C. The cells were washed and, if needed, fixed in cold acetone or isopropanol at 0°C for 12mn and studied using epifluorescent microscopy. The anionic vesicles delivered the Rh-PE to the lysosomes as determined by the identical green and red punctate fluorescent staining patterns. Rh-PE cationic vesicles in contrast gave diffuse cell staining patterns.

### EXAMPLE 5: Enhanced uptake of labelled DOPG containing vesicles when complexed with cationic vesicles.

Rhodamine labelled DOPG vesicles are anionic and when delivered to tissue cultures cells, a punctate staining pattern was observed that is indicative of endocytic uptake and lysosomal localization.

Rh-PE labelled negatively charged liposomes (2 nmol DOPG) were complexed with Lipofectin™ (10nmol DOTMA)in Opti-MEM. CV-1 cells were treated with the Rh-PE DOTMA/DOPG complexes for 4h. or 24h. at 37°C. While cultures treated with Rh-PE/DOPG alone had punctate fluorescent staining patterns consistent with lysosomal localization, DOPG/DOTMA complex treated cells had diffuse cell membrane staining consistent with fusion. The presence of cationic lipid on the surface of the vesicles permitted the anionic lipid component to bypass the lysosomal compartment. More diffuse membrane staining was observed after 24 hours than at the 4 hour incubation times.

### EXAMPLE 6: Enhanced uptake of anionic vesicles encapsulating rhodamine phalloidin or beta-galactosidase using cationic lipid vesicles

Rhodamine phalloidin and beta-galactosidase were separately encapsulated into negatively charged liposomes. Beta-galactosidase cleaves the chromogenic substrate X-gal to release a blue indoyl derivative. Beta-galactosidase at 1mg/ml was complexed with 7.5nmol DOPG or 7.5nmol DOPG with 10nmol DOTMA. The mixtures were applied to CV-1 cells for 4 hours followed by X-gal staining. Blue staining patterns were indicative of beta-galactosidase activity. Faint blue staining was observed in cultures receiving beta-galactosidase associated with DOPG vesicles. Cultures receiving beta-galactosidase with the DOPG:DOTMA vesicle complex showed intracellular cell staining.

Phalloidin is a poisonous alkaloid that stabilizes and specifically binds actin filaments and prevents their depolymerization within the cell. Phalloidin does not cross cell membranes and hence its very presence in the cell in these experiments indicates directed liposome mediated delivery.

A 3.3 M Rhodamine phalloidin solution was incorporated into anionic vesicles followed by the addition of 8nmol DOPG or 12nmol DOPG with 20nmol DOTMA. These mixtures were applied to duplicate CV-1 cultures for 4h at 37°C. Cultures receiving DOPG alone had fluorescent staining patterns similar to HPTS stained cells. The punctate pattern indicated lysosomal uptake. Cultures receiving DOPG and DOTMA vesicle complexes resulted in rhodamine fluorescent staining patterns of actin filaments.

These experiments were additionally repeated by incorporating either rhodamine phalloidin or beta-galactosidase directly into DOTMA:DOPE vesicles (direct vs. indirect complex preparation) and these experiments produced more intense membrane fluorescence.

Neither phalloidin nor beta-galactosidase are membrane bound proteins. This example illustrates that non-membrane associated proteins can be delivered to the cell cytoplasm and maintain their function. We have noted that it may be advantageous to deliver other proteins or bioactive substances to CF airway epithelium alone or in combination with CFTR protein or nucleic acid. Thus this type of strategy offers the directed delivery of protein to either the cell cytoplasm or cell membranes. An example of intracellular protein delivery using DOTMA/DOPG or DOPE vesicles is outlined below.

### EXAMPLE 7: Cholera toxin subunit A encapsulated into DOTMA/DOPE vesicles is delivered to the cytoplasm and stimulates intracellular cyclic AMP production.

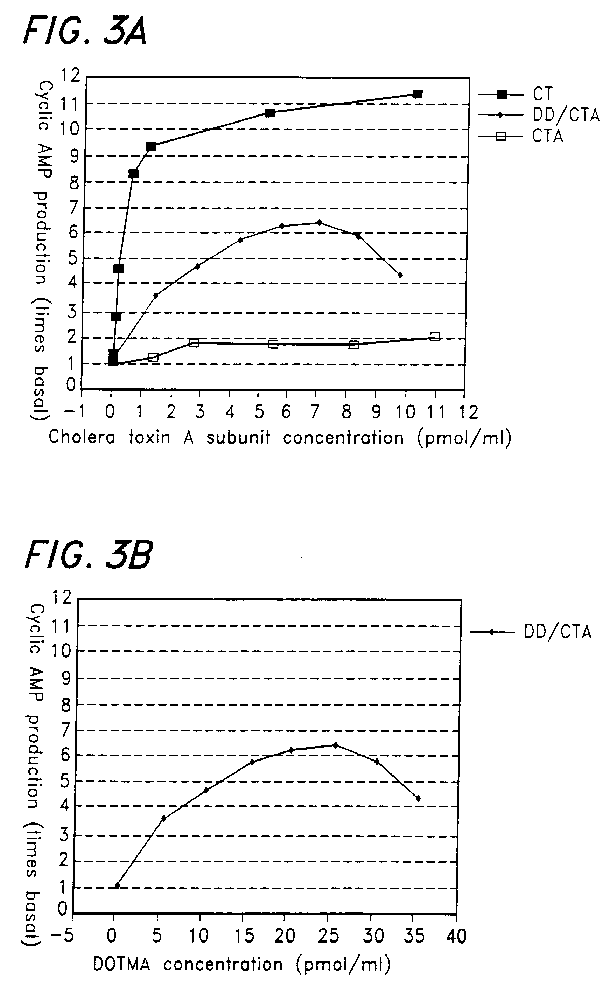

Accumulation of radioactive cyclic AMP in CEM cells was monitored following treatment with cholera toxin. Results are provided in Figure 3. The cholera toxin subunit A was encapsulated into DOTMA/DOPE lysosomes (solid line). The toxin was also encapsulated into anionic vesicles (empty squares). Cholera toxin holotoxin was used as a positive control (filled squares). Figure 3 illustrates the results using increased concentrations of cholera toxin subunit A. Results indicate that for this application the direct approach of complexing protein with lipid was more efficient than the indirect approach.

The delivery of protein and nucleic acid sequences to the airway epithelium of patients with cystic fibrosis requires effective liposome delivery to viscous mucus filled lungs. DNase I has been used to decrease the viscosity of CF mucus and in the past was used routinely. Below we offer evidence to indicate that DNase I treatment of CF lungs prior to or concomitant with liposome delivery yields unexpected advantages for bioactive molecule delivery that cannot be attributed to a reduction in mucus viscosity alone.

### EXAMPLE 8: Membrane fusion inhibition of DORIE vesicles by DNA in vitro

DORIE:DOPE vesicles were membrane labelled with Rhodamine-PE as described in Example 3. CV-1 cultures were set up in OPTI-MEM or Opti-MEM with 50 ug/ml calf thymus DNA. Cationic liposomes with Rhodamine-PE were added in duplicate to CV-1 cultures, already stained with HPTS, with or without calf thymus DNA. Cultures containing calf thymus DNA showed an overwhelming reduction of diffuse surface fluorescence (Rh-PE) and an increase in punctate surface fluorescence as compared with CV-1 cultures that did not contain calf thymus DNA. Thus exogenous DNA directly inhibited cationic liposome mediated delivery at concentrations that did not visually increase the viscosity of the culture media. An explanation for this inhibitory activity is that DNA is a large anionic molecule that binds directly to the cationic liposome and neutralizes the net positive charge thereby reducing the efficiency of liposome complex delivery.

In cystic fibrosis patients, the bronchial airways are filled with mucus containing extremely high concentrations of DNA (3-14 mg/ml). This dense mucus layer not only presents a physical barrier to liposomal delivery, but as shown here, additionally presents a potent chemical barrier.

### EXAMPLE 9: Reversal of membrane fusion inhibitory activity of DNA by DNase treatment.

The protocol for this experimental set is identical to that described in Example 8. CV-1 cells fed with HPTS were exposed to cationic liposomes comprising DORIE with Rh-PE. However in this set, all CV-1 cultures were incubated with OPTI-MEM containing 50 ug/ml calf thymus DNA. One half of the cultures were treated for 30 minutes at 37 C with 5 units of DNase I before addition of the cationic liposomes. The untreated cultures served as controls. Liposomes with Rh-PE were incubated with DNase treated and untreated cell cultures 1 hour at 37 C. Following incubation, cultures were washed and processed for fluorescent microscopy. Cultures that did not receive DNase treatment had no diffuse fluorescence only punctate membrane surface and discrete punctate lysosomal staining. Cultures receiving DNase showed diffuse membrane fluorescence with Rhodamine-PE and punctate lysosomal staining only with HPTS. These results indicated that DNase I can successfully reverse the inhibitory effect of the polynucleic acid on the fusogenicity of DORIE vesicles.

### EXAMPLE 10: Fusion of DORIE/DOPE vesicles with mouse lung bronchial epithelium in vivo.

DORIE/DOPE vesicles containing Rhodamine PE as discussed in Example 3 were prepared as noted in Example 8. Anesthetized C57BL/6 mice were intubated and 0.05 ml of the DORIE/DOPE-Rh-PE liposome formulation (50-200 nanomoles) was introduced into the trachea and allowed to drip into the bronchial airways. Twenty hours later the lungs were removed and cryostat sections of the bronchial epithelium were prepared and evaluated by epifluorescence microscopy. Sections were later stained with hematoxylin and eosin to identify targeted objects in the lung. Lung sections showed intense rhodamine fluorescent staining of the bronchial epithelial cell surfaces indicating that cell membrane delivery can occur *in vivo.*

### EXAMPLE 11: Reporter gene expression in mouse lung following introduction of DNA coding for beta-galactosidase.

Cationic liposomes were prepared containing DNA coding for beta-galactosidase. For these experiments the beta-galactosidase gene replaced the luciferase gene as diagramed in Figure 2. DNA was prepared from the construct using methods disclosed in the section entitled Preparation of DNA. DORIE:DOPE were prepared using methods disclosed in the section entitled Example 1: Positively charged liposome preparation. Saline, DNA, liposomes and DNA-liposome complexes were prepared as follows: 1. Saline 0.9% sodium chloride. 2. DNA only: 220 µl of pRSVLacZ 1.11 mg/ml in saline was diluted with 280 µl sterile water. 3. Lipid only: 390 µl 6.04 mM DORIE: 6.04 mM DOPE in sterile water ("DORIE:DOPE") was diluted with 110 µl sterile water. 4. DNA:Liposome 1: 220 µl of pRSVLacZ 1.11 mg/ml in saline was diluted with 280 µl of DORIE:DOPE (cationic lipid:DNA molar ratio is 2.2). 5. DNA:Liposome 11: 110 µl of pRSVLacZ 1.11 mg/ml in saline was diluted with 390 µl DORIE:DOPE (cationic lipid:DNA molar ratio 6.24).

About 100 µl of each solution was introduced into the trachea of metophane-anesthetized C57B1/6 mice. Intratracheal administration was carried out using a glass hypodermic BD Yale syringe and Minicath 19G 7/8th inch o.d. tubing (Cat. No. 1912 Deseret Medical Co.). Intratracheal administration was verified by chest palpation. Total DNA/DORIE/DOPE doses in µg per 100 µl per animal were, respectively 1.= 0/0/0; 2.= 49/0/0; 3.= 0/340/350; 4.= 49/245/251; 5.= 24/340/350.

Lungs were collected uninflated at 2 days (1 animal each) and 4 days (2 animals each group) post-injection, frozen at - 150°C in isopentane, embedded in O.C.T. and sectioned in a cryostat at -18°C. Serial 15 µm sections were collected, melted on glass slides. The sections were dried and every 10th section was stained overnight at 37°C histochemically for β-galactosidase enzyme activity (functional LacZ gene product) using a standard X-Gal reaction mixture (sodium phosphate, magnesium chloride, sodium ferricyanide, sodium ferrocyanide, and X-gal) and then counterstained with hematoxylin-eosin (H&E). Adjacent sections were stained only for H&E. Specific β-galactosidase gene product was visualized in successfully transfected cells by microscopy as an intense blue cytoplasmic reaction product.

The results indicated that normal, saline-, DNA- and lipid-injected mouse lung contains some punctate and rod-shaped X-Gal staining within interstitial and airway cells. Occasional X-Gal positive cytoplasmic staining of interstitial cells was additionally observed. However, lipid-DNA-injected lungs contained the above positive staining and in addition an extensive cytoplasmic staining of airway cells over extensive regions of bronchial lining.

### EXAMPLE 12: Treatment of CF Patient with DNase, Cationic Lipid/Polynucleotide or Protein Complex

For initial studies, a CF patient is chosen for CFTR therapy that is clinically stable, i.e. having had no hospitalizations or changes in medication in the last month.

The patient is initially treated daily or twice daily with a composition of fluid deliverable as a spray from a hand-held nebulizer since small droplet size needed for deep lung penetration is not necessary for this application. Fluid delivery is intended for the airway epithelial cells. DNase I may preferably be delivered alone until mucus viscosity is decreased or together with the therapeutic macromolecule complexed with lipid. A dose of 2.5 ml (preferably in a range from 0.25 ml to 25 ml.) containing 2.5 mg of mRNA (preferably a range of 0.1-20 mg/ml), 5 mg CFTR protein (between 0.1-10 mg/ml) or both, together with the appropriate lipids to form cationic complexes that are fusogenic with cell membranes and, if needed, 3 ug/ml DNase I (between 1.0ug/ml-10mg/ml) is loaded into the nebulizer and inhaled. The diluted polynucleotide, protein, DNase I and liposome solutions are prepared from concentrated stocks by dilution into a buffer suitable for lung inhalation (for example 0.9% NaCl). These therapies can be repeated on a regular basis until clinical improvement is observed.

The exact dosage regime may be varied both in frequency and in quantity to benefit a given patient. It is anticipated that this regime will be optimized during animal trials and perfected during clinical trials in accordance with conventional evaluation techniques.

To promote maximal DNase I activity, particularly in the cationic liposome complex formulation, the enzyme can be provided to the patient in a supportive buffer containing manganese and magnesium ions to promote maximal nuclease activity.

The patients will receive daily or twice daily doses of the CFTR gene, by aerosol directly into the lung. The target bronchial epithelial cells can be reached with the spray from a simple hand-bulb nebulizer. The liposome complexes alone or with DNase I, as necessary, is provided to maintain therapeutic efficacy.

Efficacy will be determined by assaying for the presence of the CFTR gene product in a bronchial epithelial cell scraping from treated cystic fibrosis patients or for the presence of CFTR protein using immune histochemical techniques. Further, patients will be clinically assessed for improvements in lung function. This assessment can include viscoelastic measurements of sputum, improvements in pulmonary function including improvements in forced expiratory volume and maximal midexpiratory flow rate. If antibiotics are coadministered as part of the patient therapy, bacterial quantitation following therapy can be included. Pulmonary function tests as well as diagnostic tests for the clinical progression of CF are well known to those individuals with skill in this art.

It is further contemplated that the invention disclosed herein can be formulated into a kit. The kit may contain one or more containers to hold dosages of DNase, the therapeutic molecule (CFTR nucleic acid, CFTR protein, other nucleic acids or proteins or other bioactive substances causally related to CF) either alone or complexed with the lipids of choice. An apparatus for the delivery of the formulation is additionally contemplated. This apparatus is preferably a hand-held nebulizer or the like, but could also be a syringe like device or other suitable device for tracheal instillation, or a more complex aerosolization apparatus such as a Centimist nebulizer (Intec Corp., Blue Springs, MO). Apparatus for connecting the containers to conventional breathing or atomizing apparatus and for introducing the compositions into the patient through such apparatus are also contemplated. The apparatus comprises one or more containers to hold the pharmaceutical composition that includes a macromolecule to remedy the cellular defect associated with cystic fibrosis, cationic lipid suitable for delivery of the macromolecule with or without DNase. The kits, including a suitable apparatus, may be packaged with varying dosages for various age groups and may include single or multiple doses.

## Claims

1. A pharmaceutical composition suitable for pulmonary administration comprising therapeutically effective amounts of:
DNase suitable for reducing the viscosity of pulmonary mucus in a cystic fibrosis patient;
a macromolecule that provides functional polypeptide to remedy the cellular defect associated with cystic fibrosis, whereby the macromolecule comprises a polynucleotide sequence operatively coding for said functional polypeptide or comprises a functional polypeptide; and
an amount of cationic lipid effective to deliver said macromolecule into pulmonary cells in vivo.

2. The composition of claim 1 wherein the amount of polynucleotide is sufficient to encode a therapeutically efficacious amount of polypeptide.

3. The composition of claim 1 or 2 wherein said polynucleotide encodes the cystic fibrosis transmembrane conductance regulator (CFTR).

4. The composition of claim 1, 2 or 3 wherein said polynucleotide is DNA or mRNA.

5. The composition of claim 4 wherein said mRNA lacks a 5'capping nucleotide.

6. The composition of any one of claims 1 to 5 wherein said composition is in the from of an aerosol or in a form suitable for delivery via pulmonary instillation.

7. The composition of claim 1 wherein said lipid and said polypeptide form a cationic complex.

8. The composition of claim 1 wherein the amount of polypeptide is therapeutically efficacious.

9. The composition of claim 1, 7 or 8 wherein said polypeptide is the cystic fibrosis transmembrane conductance regulator (CFTR).

10. The composition of any one of claims 1 to 9 wherein said DNase is a recombinant protein.

11. The composition of claim 1 wherein said DNase is human DNase I.

12. The use of a composition according to any one of claims 1 to 11, for the manufacture of a medicament for treating a patient having cystic fibrosis.

13. The use of claim 12 wherein said medicament comprises DNase as a separate composition from said macromolecule and said cationic lipid.

14. The use of claim 12 wherein said medicament comprises effective amounts of said macromolecule and said cationic lipid in a single composition.

15. An apparatus for the treatment of cystic fibrosis, comprising:
a container housing therapeutically effective amounts of the composition of any one of claims 1 to 11; and a delivery device associated with said container to facilitate delivery of said composition to the pulmonary system.

16. The apparatus of claim 15 wherein said delivery device is adapted to deliver said composition as an aerosol or by pulmonary instillation.

17. A process for the preparation of a pharmaceutical composition according to any one of claims 1 to 11 which comprises combining the DNase, the macromolecule and the cationic lipid.

18. A kit useful for treating cystic fibrosis patients, comprising separate containers housing effective amounts of the components of the composition as claimed in any one of claims 1 to 11.

## Patentansprüche

1. Arzneimittel, das zur pulmonalen Verabreichung geeignet ist, umfassend therapeutisch wirksame Mengen an:
DNase, die geeignet ist, um die Viskosität von Lungenschleim bei einem Patienten mit cystischer Fibrose zu verringern;
einem Makromolekül, das ein funktionelles Polypeptid zur Behebung des zellulären Defekts bereitstellt, der mit cystischer Fibrose assoziiert ist, wobei das Makromolekül eine Polynucleotidsequenz, die das funktionelle Polypeptid codiert, oder ein funktionelles Polypeptid umfaßt; und
eine Menge an kationischem Lipid, die wirksam ist, um das Makromolekül in vivo in Lungenzellen einzubringen.

2. Mittel nach Anspruch 1, wobei die Menge an Polynucleotid ausreicht, um eine therapeutisch wirksame Menge an Polypeptid zu codieren.

3. Mittel nach Anspruch 1 oder 2, wobei das Polynucleotid den Cystische Fibrose-Transmembranleitfähigkeitsregulator ("cystic fibrosis transmembrane conductance regulator"; CFTR) codiert.

4. Mittel nach Anspruch 1, 2 oder 3, wobei das Polynucleotid DNA oder mRNA ist.

5. Mittel nach Anspruch 4, wobei die mRNA kein 5'-Capnucleotid aufweist.

6. Mittel nach einem der Ansprüche 1 bis 5, wobei das Mittel in Form eines Aerosols vorliegt oder in einer Form, die die Verabreichung durch pulmonale Instillation erlaubt.

7. Mittel nach Anspruch 1, wobei das Lipid und das Polypeptid einen kationischen Komplex bilden.

8. Mittel nach Anspruch 1, wobei die Menge an Polypeptid therapeutisch wirksam ist.

9. Mittel nach Anspruch 1, 7 oder 8, wobei das Polypeptid der Cystische Fibrose-Transmembranleitfähigkeitsregulator ("cystic fibrosis transmembrane conductance regulator"; CFTR) ist.

10. Mittel nach einem der Ansprüche 1 bis 9, wobei die DNase ein rekombinantes Protein ist.

11. Mittel nach Anspruch 1, wobei die DNase DNase I vom Menschen ist.

12. Verwendung eines Mittels nach einem der Ansprüche 1 bis 11 zur Herstellung eines Medikamentes zur Behandlung eines Patienten mit cystischer Fibrose.

13. Verwendung nach Anspruch 12, wobei das Medikament DNase als separates Mittel neben dem Makromolekül und dem kationischen Lipid umfaßt.

14. Verwendung nach Anspruch 12, wobei das Medikament wirksame Mengen des Makromoleküls und des kationischen Lipids in einer einzigen Zusammensetzung umfaßt.

15. Gerät zur Behandlung cystischer Fibrose, umfassend: einen Behälter, der therapeutisch wirksame Mengen des Mittels nach einem der Ansprüche 1 bis 11 enthält; und eine mit dem Behälter verbundene Verabreichungsvorrichtung zur Erleichterung der Verabreichung des Mittels an das Lungensystem.

16. Gerät nach Anspruch 15, wobei die Verabreichungsvorrichtung für die Verabreichung des Mittels als Aerosol oder durch pulmonale Instillation angepaßt ist.

17. Verfahren zur Herstellung eines Arzneimittels nach einem der Ansprüche 1 bis 11, bei dem die DNase, das Makromolekül und das kationische Lipid miteinander kombiniert werden.

18. Kit, geeignet zur Behandlung von Patienten mit cystischer Fibrose, umfassend getrennte Behälter, die wirksame Mengen der Bestandteile des Mittels nach einem der Ansprüche 1 bis 11 enthalten.

## Revendications

1. Une composition pharmaceutique appropriée pour une administration par voie pulmonaire, comprenant des quantités efficaces du point de vue thérapeutique de:
une DNase appropriée pour réduire la viscosité du mucus pulmonaire chez un patient souffrant de mucoviscidose;
une macromolécule qui fournit un polypeptide fonctionnel pour remédier au défaut cellulaire associé à la mucoviscidose, la macromolécule comprenant une séquence polynucléotidique codant pour ledit polypeptide fonctionnel ou comprenant un polypeptide fonctionnel; et
une quantité d'un lipide cationique efficace pour délivrer ladite macromolécule dans les cellules pulmonaires in vivo.

2. La composition selon la revendication 1, dans laquelle la quantité de polynucléotide est suffisante pour coder pour une quantité efficace du point de vue thérapeutique du polypeptide.

3. La composition selon la revendication 1 ou 2, dans laquelle ledit polynucléotide code pour le régulateur de la conductance transmembranaire de la mucoviscidose (CFTR).

4. La composition selon la revendication 1, 2 ou 3 dans laquelle ledit polynucléotide est un ADN ou un ARNm.

5. La composition selon la revendication 4, dans laquelle ledit ARNm ne comporte pas un nucléotide de la coiffe en 5'.

6. La composition selon l'une quelconque des revendications 1 à 5, dans laquelle ladite composition est sous la forme d'un aérosol ou sous une forme appropriée pour une libération par instillation pulmonaire.

7. La composition selon la revendication 1, dans laquelle ledit lipide et ledit polypeptide forment un complexe cationique.

8. La composition selon la revendication 1, dans laquelle la quantité de polypeptide est thérapeutiquement efficace.

9. La composition selon la revendication 1, 7 ou 8, dans laquelle ledit polypeptide correspond au régulateur de la conductance transmembranaire de la mucoviscidose (CFTR).

10. La composition selon l'une quelconque des revendications 1 à 9, dans laquelle ladite DNase est une protéine recombinante.

11. La composition selon la revendication 1, dans laquelle ladite DNase est une DNase I humaine.

12. L'utilisation d'une composition selon l'une quelconque des revendications 1 à 11, pour la préparation d'un médicament pour le traitement d'un patient souffrant de mucoviscidose.

13. L'utilisation selon la revendication 12, dans laquelle ledit médicament comprend une DNase en tant que composition distincte de ladite macromolécule et dudit liquide cationique.

14. L'utilisation selon la revendication 12, dans laquelle ledit médicament comprend des quantités efficaces de ladite macromolécule et dudit lipide cationique dans une composition unique.

15. Un appareil pour le traitement de la mucoviscidose, comprenant:
un récipient renfermant des quantités efficaces du point de vue thérapeutique de la composition selon l'une quelconque des revendications 1 à 11; et
un dispositif de libération associé audit récipient pour faciliter la libération de ladite composition dans le système pulmonaire.

16. L'appareil selon la revendication 15, dans lequel ledit dispositif de libération est adapté pour délivrer ladite composition sous la forme d'un aérosol ou par instillation pulmonaire.

17. Un procédé de préparation d'une composition pharmaceutique selon l'une quelconque des revendications 1 à 11, qui comprend la combinaison de la DNase, de la macromolécule et du lipide cationique.

18. Un kit utile pour le traitement des patients souffrant de mucoviscidose, comprenant des récipients séparés renfermant des quantités efficaces des composants de la composition selon l'une quelconque des revendications 1 à 11.
